# EUROPEAN PATENT APPLICATION

(11) **EP 2 562 665 A2**
(43) Date of publication of application: **27.02.2013**
(21) Application number: 12167991.4
(22) Date of filing: 15.05.2012
(51) Int. Cl.: G06F 19/00

(54) **Clinical platform**

(30) Priority: 16.05.2011 ES 201130789
(71) Applicant: Gomez Borrallo, Juan Jose, 28028 Madrid (ES)
(72) Inventor: Gomez Borrallo, Juan Jose, 28028 Madrid (ES)
(74) Representative: Isern-Jara, Nuria

(57) **Abstract**

The platform of the invention is constituted starting from CPA "Access Profesional Cloud" professional access to a cloud in which said access is carried out by means of a device or main server (1), having a particular software, developed within the frame of the cloud computation services, through which it is possible the access to different applications and services such as communication with hospitals (2), medical applications (3), medical databases of images (4), communication with surgery teams (5), communications doctor-patient (6), medical equipment (7), and similar. To this server each the different hospitals (8), medical firms (9) and the company (32) in charge of managing of the platform (10) are connected, to which server the previously register users may have, being said users doctors or patients by means of the creation of customized profile for each user by means of which the services or applications to which the user may access will be discriminated. The access may be carried out through computers (10), mobile telephones (11) or specially devised apparatus (12).

## Description

### Object of the invention

The present invention refers to a clinical-simulation platform that is, a platform which permits the access to different applications such as systems for clinical training, visualization systems, doctor/patient communication systems, as well as the access to other applications.

The object of the invention is to provide an interface to gain access to an extremely versatile clinical-simulation platform, easy to use, which permits a customized treatment, both personal as professional.

The invention corresponds therefore to the clinical field.

### Background to the invention

As is well known, in the field of the practical application of the invention and particularly in the doctor/patient environment, the patient must physically visit the doctor in order to obtain a diagnosis, the doctor having available a data base to store and consult information about each patient.

Although the doctor/patient communication by means of computation services through Internet®, by computers and the corresponding web-cams is well known, this type of communication is very limited due to the fact that no platform exists to add up this type of applications to other usual and/or necessary applications within the clinical field. Additionally, access and authentification problems for the users have to be taken into consideration, as well as the fact of not having available an interface easy to use to gain access to said services.

### Description of the invention

The platform provided by the invention has as objective to fill up the above explained void, providing a platform to permit the access to different applications, such as clinical trainining systems, visualization systems, doctor/patient communication systems, as well as other applications, all of it by means of an extremely versatile access interface, easy to use, which permits customized treatment, both personal and professional.

To this end and in a particular form, the platform which is the subject matter of the invention is implemented starting from CPA "Access Professional Cloud" (professional access to the Internet web), said access being implemented through a device associated to a complementary software, developed within the frame of the cloud computation services by means of Internet, through which the access is possible to different applications so that said applications and the platform are located in the cloud as those above cited, which lends a possibility of access from hospitals, medical societies, the company in charge of the management of the platform, as well as independently to different users, both doctors or patients, the creation of a customized profile having been foreseen for each user by means of which the services or applications to which the user may have access will be discriminated.

Particularly, it has been foreseen that the access to the different systems/applications associated to the platform may be carried out only through a user registration, in which the profile of each user is defined.

According to said profile, a control center or main server will be able to choose the content or applications which are adequate to the specific peculiarities of the user.

Therefore, the registration implies the activation of a personal account in said main server.

The platform, being in connection with different centers and organizations, will be continuously updated with new applications or services sending information about the same to the adequate users in function of its profile, so that these may enter a contract for said new services in case that they are not free, through the control center or main server.

Concerning the means to access the platform while the users of the same will have capacity of access through a computer, telephone having navigation means through Internet or similar, a specific device has been provided for that function, being implemented in a thin rectangular housing having in its front side a screen extending to the majority of its surface, consisting in a touch screen associated to a microprocessor, with its corresponding power source and wireless connecting means to Internet®, which will be supplied with one or two touch pencils having double click push button, the device incorporating a push button array with the basic functions, in its front face as well as in the side faces of the housing, among which an on/off push button, a push button for the screen configuration, a push button for blocking the screen, a push button for runback, a push button to access the main menu and push buttons for the regulation of the volume.

Simultaneously, a device is being provided to incorporate a SD card reader, SIM card for access to the Internet, an audio connector, as well as means for transmission/reception of wireless data, such as Bluetooth and Wifi.

Lastly, it is to be remarked that this device incorporates in its front side, as well as in its rear side, at least one digital camera.

### Description of the drawings

To complete this description and in order to assist a better understanding of the features of the invention, according to a preferred embodiment of the same, a set of drawings is being accompanied to this description, forming part of the same, and having only illustrative and non limitative character, in which:
Figure 1 shows a block diagram of a clinical-simulation platform according to the present invention.
Figure 2 shows a perspective view of the device provided as specific means to access the platform of the invention.
Figure 3 shows a side view of the device shown in figure 2.
Figure 4 shows a rear elevation view of the device in figure 2.
Figure 5 shows a plant view of the device in figure 2.
Figure 6 shows a lower plant view of the device in figure 2.
Figure 7 shows a plant view of two auxiliary means which complete the device in figure 2, particularly two touch pencils.

### Preferred embodiment of the invention

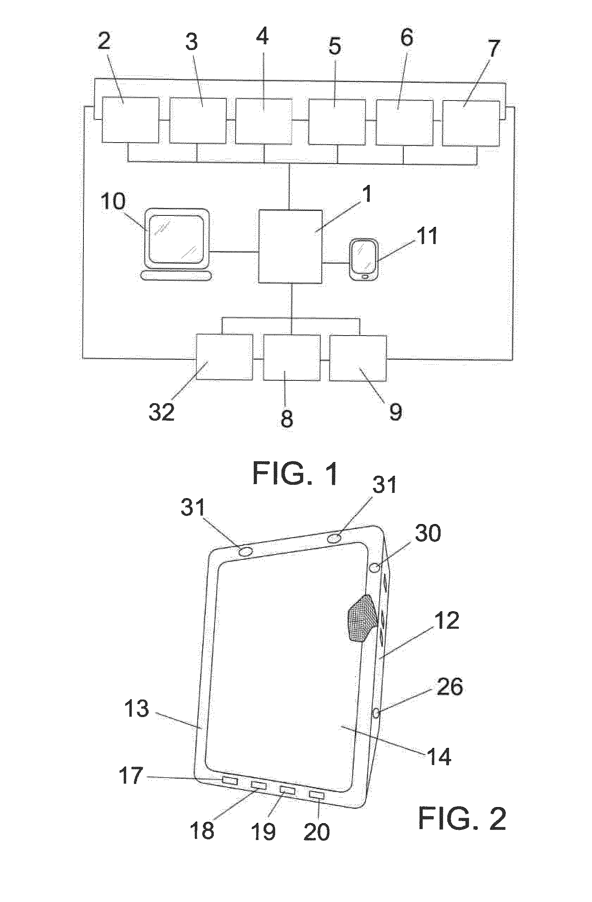

Referring to the above cited figures, and particularly to figure 1, it may be observed that the invention is based on CPA "Access Professional Cloud", professional access to cloud in which said access is carried out by means of a device or main server (1), having a particular software, developed within the frame of the cloud computation services, which main server (1) permits the access to different applications and services such as communication with hospitals (2), medical applications (3), medical databases of images (4), communication with surgery teams (5), communications doctor-patients (6), medical equipment (7), etc.; to which server the different hospitals (8), medical firms (9) as well as the company (32) in charge of managing the platform are connected to the server. Likewise, the previously registered users, for instance doctors or patients, may have access through the server to the computers (10), mobile telephones (11) or specially devised apparatus (12), as that shown in figures 2 to 7.

As above stated, to have access to the platform it is necessary to create a customized profile for each user by means of which the services or applications to which the user may have access will be discriminated.

According to said profile, the control center or main server (1) will permit to choose the contents or applications (2-7) which are more adequate to the specific peculiarities of the user.

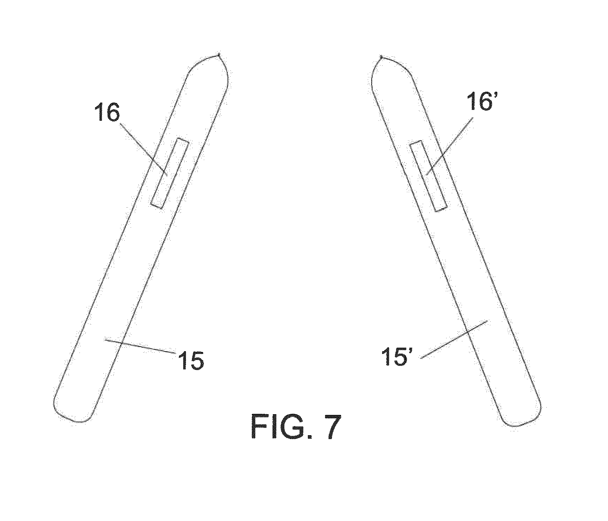

The device to access the platform, specially arranged according to the invention, has an rectangular housing (13), of the reduced thickness, having on its front side a touch screen (14), with its corresponding microprocessor, power source and wireless means for connection to Internet®, being completed with a couple of touch pencils (15, 15'), shown in detail in figure 7, having a double click push button (16, 16')

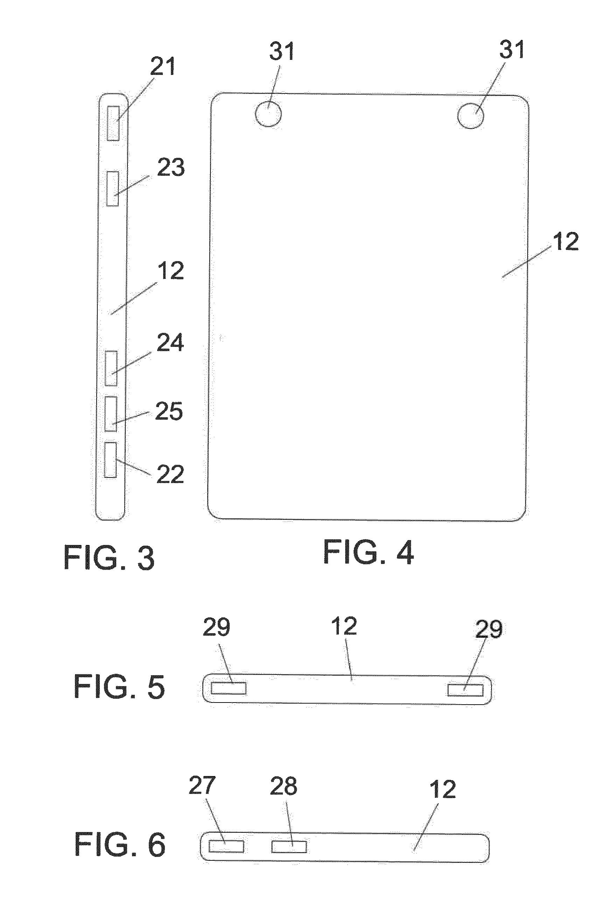

As is to be seen in figure 2, the device incorporates in its front side a command for the activation of the menu (17), a push button for the configuration of the screen (18), a push button for blocking the screen (19), and an runback push button (20), while on one of its side edges, as shown in figure 3, the device incorporates on/off push button (21) and a command for the regulation of the volume (22), as well as a SD card reader (23), an UMD card reader (24), and an USB port (25).

In the opposite side the device incorporates an audio connector (26), while in the lower base a SIM card reader (27) is incorporated, and an additional data transmission port (28) may be also incorporated in said lower base.

As above stated, the device has wireless means for data transmission/reception as Bluetooth (29) and Wifi (30), as well as digital cameras (31) incorporated in its front and rear sides.

## Claims

1. Clinical-simulation platform, **characterized in that** it is constituted starting from CPA "Access Professional Cloud" professional access to cloud in which said access is carried out by means of a device or main server (1), having a particular software, developed within the frame of the cloud computation services, enabling said main server (1) the access to different applications and services such as communication with hospitals (2), medical applications (3), medical databases of images (4), communication with surgery teams (5), communications doctor-patient (6), medical equipment (7), and similar, to which server the different hospitals (8), medical firms (9) and the company (32) in charge of managing of the platform are connected, to which server the previously registered users having capacity of access to the server by means of the creation of customized profile for each user, by means of which will be discriminated the services or applications to which the user may access, whether doctors or patients by means of computers (10), mobile telephones (11) or specially devised apparatus (12).

2. Clinical-simulation platform, according to the claim 1, **characterized in that** the means (12) especially devices for the access to the platform, comprise a device formed by a rectangular housing (13), with a reduced thickness, having in its front side a touch screen (14), with its corresponding microprocessor, power source and wireless means for connection to Internet®, being completed with a least one touch pencil (15), which device has a push button array for basic control functions, as well as a SD card reader (23), an UMD card reader (24), an USB port (25), an audio connector (26), a SIM card reader (27), as well as wireless means for data transmission/reception, such as Bluetooth (29) and Wifi (30), as well as digital cameras (31) in each of its front and rear sides.

3. Clinical-simulation platform, according to the claim 2, **characterized in that** the touch pencil (15) has a double click push button (16).
